(19) Europäisches Patentamt
European Patent Office
Office européen des brevets

(11) **EP 1 632 577 A2**

(12) **EUROPEAN PATENT APPLICATION**

(43) Date of publication:
**08.03.2006 Bulletin 2006/10**

(51) Int Cl.:
***C12Q 1/26*** (2006.01)

(21) Application number: **05007051.5**

(22) Date of filing: **31.03.2005**

(84) Designated Contracting States:
**AT BE BG CH CY CZ DE DK EE ES FI FR GB GR
HU IE IS IT LI LT LU MC NL PL PT RO SE SI SK TR**
Designated Extension States:
**AL BA HR LV MK YU**

(30) Priority: **01.09.2004 JP 2004254709**

(71) Applicant: **FUJITSU LIMITED**
**Kawasaki-shi,**
**Kanagawa 211-8588 (JP)**

(72) Inventors:
• **Tomanaga, Atsushi**
**Fujitsu Limited**
**Kawasaki-shi**
**Kanagawa 211-8588 (JP)**

• **Shiobara, Noriyuki**
**Fujitsu Limited**
**Kawasaki-shi**
**Kanagawa 211-8588 (JP)**
• **Ueda, Akihiko**
**Fujitsu Limited**
**Kawasaki-shi**
**Kanagawa 211-8588 (JP)**

(74) Representative: **Sunderland, James Harry**
**Haseltine Lake,**
**Imperial House,**
**15-19 Kingsway**
**London, WC 2W 6UD (GB)**

(54) **Apparatus, method, and computer product for supporting estimation of metabolism**

(57) Structure information of an estimation target compound and structure information of an arbitrary unit of CYP are acquired. Stable position information on a stable position in which the estimation target compound is stably disposed in a pocket of the CYP based on the acquired structure information. A distance between an atom of the estimation target compound that is stably positioned in the pocket and heme iron of the CYP is calculated based on the acquired stable position information. Whether the estimation target compound is to be a substrate of the CYP is determined based on the calculated distance.

**EP 1 632 577 A2**

**Description**

CROSS-REFERENCE TO RELATED APPLICATIONS

This application is based upon and claims the benefit of priority from the prior Japanese Patent Application No. 2004-254709, filed on September 1, 2004, the entire contents of which are incorporated herein by reference.

BACKGROUND OF THE INVENTION

1) Field of the Invention

The present invention relates to a technology for supporting estimation of metabolism.

2) Description of the Related Art

A drug that enters a body is treated as a substance foreign to the body, and is gradually transformed to a substance that is more water-soluble through various reactions inside the body to be excreted from the body. This transformation is called metabolism, and the most important catalyst for metabolic reaction is cytochrome P450. Cytochrome P450 (abbreviated to "CYP", and is classified in a format CYPnXm, where n and m are natural number, and X is a capitalized alphabet, hereinafter, "CYP") is a general term for about a hundred types of enzymes to be a catalyst for various metabolic reactions (mainly oxidation reaction) by activating oxygen. It is a common knowledge that variation in metabolic reaction speed with CYP directly affects the effectiveness of a drug, duration of the effect of the drug, and difference in degree of side effect among individuals.

A series of intermediary substances that are produced by the metabolic reaction to the drug temporarily accumulates in the body. Therefore, it is essential for drug development to comprehensively follow up information on the intermediary substance, such as what kind of substance the intermediary substance specifically is, how much amount is produced, whether the intermediary substance can be a factor of a side effect, and whether the intermediary substance is toxic. Moreover, it is important information whether the drug is to be a substrate or an inhibitor of CYP for understanding drug interaction.

Conventionally, a search system for searching for similar cases of the metabolic reaction is known. The similar cases are searched by searching a partial structure from a CYP-related-metabolism information database in which cases of metabolic reaction with CYP are collected. For example, a search system disclosed in Fujitsu Limited, "BioFrontier for Windows Ver1.0 database software for information on metabolites and inhibitors", online, 2001, Fujitsu, [searched on August 2, 2004], the Internet <URL:
http://venus.netlaboratory.com/material/messe/biofrontier/>
stores about 3700 cases as of now.

In the above search system, the CYP-related-metabolism information database is constructed by collecting transformation patterns of a portion involved in the metabolic reaction based on the metabolic reaction cases collected. The transformation pattern is called transformation. In the search system, transformation of a compound of which metabolism is to be estimated is searched. It is possible to obtain candidate metabolites by applying a structural change after the metabolic reaction to the compound. In the search system disclosed in non patent literature 1 described above, about 1000 types of transformation can be applied as of now.

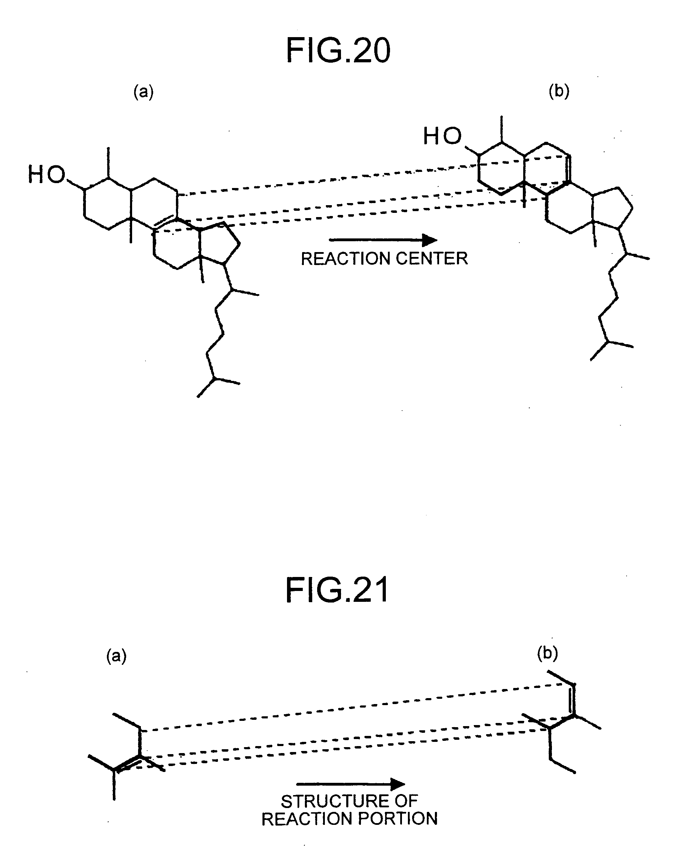

The search system described above is briefly explained below. First, a structure data of a reaction substrate and structure data of a reaction product in enzyme reaction of each type of CYP are obtained from known metabolism information data relating to CYP that is stored in the CYP-related-metabolism information database. Then, a reaction center in known metabolic reaction relating to each CYP is calculated. The "reaction center" is an atomic pair of which a chemical environment changes after reaction among corresponding atomic pairs in a structure of the reaction substrate and a structure of the reaction product. Fig. 20 is a schematic for explaining a concept of the reaction center in the known metabolic reaction. In Fig. 20, (a) illustrates the structure of the reaction substrate, and (b) illustrates the structure of the reaction product. A portion connected with a broken line between (a) and (b) in Fig. 20 is an atom (reaction center atom) to be the reaction center.

Then, atoms that can be traced within a predetermined bonding number from each reaction center atom are extracted to acquire a structure of a reaction portion. Fig. 21 is a schematic for explaining a concept of the structure of the reaction portion. In the example shown in Fig. 21, an atom that can be traced within the predetermined bonding number (the bonding number is one in the example shown in Fig. 21) from the reaction center atom shown with a broken line is extracted, and the structure of the reaction portion ((a) shown in Fig. 21) of the reaction substrate and the structure of the reaction portion ((b) shown in Fig. 21) of the reaction product are acquired. Removing the broken line from the structure of the reaction portion, a reaction formula is obtained. The reaction formula is called "transformation formula" (or just "transformation").

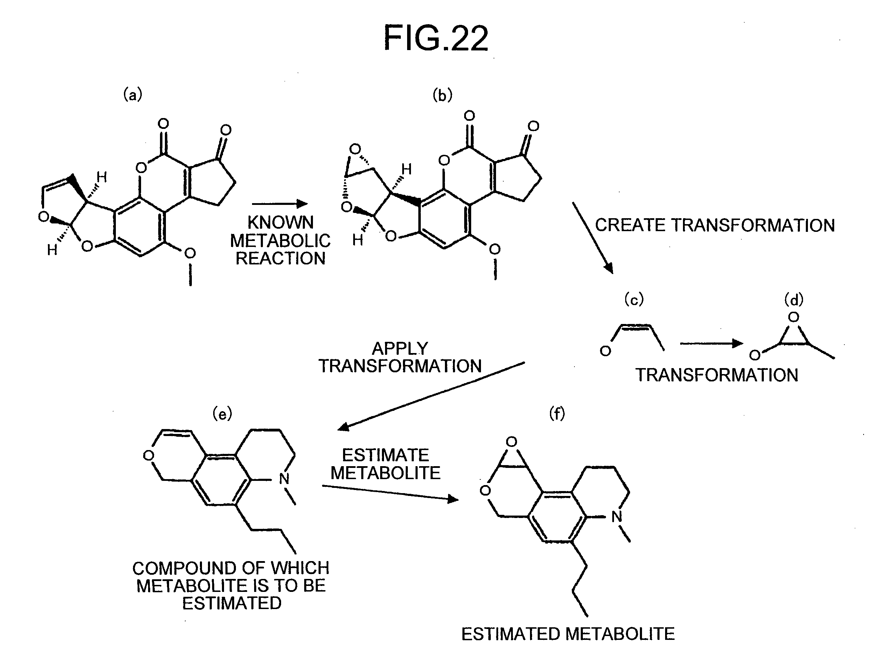

Fig. 22 is schematic for explaining the concept of the estimation of metabolism performed by applying transformation that is created by the known metabolic reaction relating to each CYP to a compound of which a metabolite is to be estimated. In Fig. 22, (a) illustrates the structure of the reaction substrate, and (b) illustrates the structure of the reaction product. The reaction center is acquired by comparing these two structures. The atoms that can be traced within the predetermined bonding number (the bonding number is one in the example shown in Fig. 22) from each reaction center atom are extracted. Thus, the transformation is created ((c) and (d) shown in Fig. 22).

When the left side (reaction substrate side) of the transformation formula is included in a structure of a compound, which is an estimation target, the transformation is applicable. The transformation means that recombination occurs after reaction. Therefore, if a structure in the right side (reaction product side) of the transformation formula is applied to the compound, it is possible to create a structure of an estimated metabolite.

In other words, when a process of a metabolism, from the known reaction to creation of the estimated metabolite, is viewed in terms of a structural formula, applicability of a transformation formula to a target compound is determined based on a structure ((c) shown in Fig. 22) in the left side of each of a great number of transformation formulas that are obtained based on various known metabolic reaction, and a structure ((d) shown in Fig. 22) in the right side of a transformation formula determined to be applicable is applied to the target compound. Thus, many possible metabolites ((f) shown in Fig. 22) can be created.

However, in a conventional technology disclosed in the literature mentioned above, even if many similar metabolic reaction cases are obtained, it is almost impossible to narrow down and evaluate 1000 cases of transformation. Therefore, it is difficult to estimate which metabolic reaction case is actually applied.

In other words, when there is more than one partial structure to which transformation is applicable in the compound to be the estimation target, it is necessary to apply many cases of transformation to each partial structure. As a result, many structures of estimated metabolites need to be created. Thus, applicability of the transformation is only based on whether matching partial structures exist. Therefore, some of the structures of the estimated metabolites and the transformation applied do not include the metabolic reaction, and it is difficult to accurately estimate the structures of the estimated metabolites obtained actually through the metabolic reactions.

Furthermore, in the conventional technology disclosed in the literature mentioned above, it is impossible to estimate whether the estimation target compound and the intermediary substances obtained in the metabolic reaction of the estimation target compound are to be the substrate or the inhibitor. Therefore, the number of drops due to the drug metabolism cannot be decreased in clinical trials in the drug development. As a result, a developing period cannot be shortened, and developing cost cannot be decreased.

## SUMMARY OF THE INVENTION

It is an object of the present invention to at least solve the problems in the conventional technology.

According to an aspect of the present invention, an apparatus for supporting estimation of metabolism includes a structure-information acquiring unit that acquires structure information of an estimation target compound and structure information of an arbitrary unit of cytochrome that includes heme iron; a stable-position-information acquiring unit that acquires stable position information that includes information on a stable position at which the estimation target compound is stably disposed in a pocket of the cytochrome based on the structure information of the estimation target compound and the structure information of the cytochrome that are acquired by the structure-information acquiring unit; a distance calculating unit that calculates a distance between an atom that is stably disposed in the estimation target compound in the pocket and the heme iron of the cytochrome based on the stable position information acquired by the stable-position-information acquiring unit; and a determining unit that determines whether the estimation target compound is to be a substrate of the cytochrome based on at least the distance calculated by the distance calculating unit.

According to another aspect of the present invention, a method for supporting estimation of metabolism includes acquiring structure information of an estimation target compound and structure information of an arbitrary unit of cytochrome that includes heme iron; acquiring stable position information that includes information on a stable position at which the estimation target compound is stably disposed in a pocket of the cytochrome based on the structure information acquired; calculating a distance between an atom that is stably disposed in the estimation target compound in the pocket and the heme iron of the cytochrome based on the stable position information acquired; and determining whether the estimation target compound is to be a substrate of the cytochrome based on the distance calculated.

According to still another aspect of the present invention, a computer-readable recording medium stores therein a computer program for realizing the above method on a computer.

The other objects, features, and advantages of the present invention are specifically set forth in or will become apparent from the following detailed description of the invention when read in conjunction with the accompanying drawings.

BRIEF DESCRIPTION OF THE DRAWINGS

Fig. 1 is a block diagram of a hardware configuration of an apparatus for supporting estimation of metabolism according to an embodiment of the present invention;

Fig. 2 is a block diagram of a functional configuration of the apparatus for supporting estimation of metabolism according to the embodiment;

Fig. 3 is a schematic for explaining three-dimensional structure data of CYP2C9;

Fig. 4 is a schematic for explaining three-dimensional structure data of CYP in which a drug is disposed;

Fig. 5 is a schematic for explaining two-dimensional structure data (a chemical reaction formula) of testosterone, which is an estimation target compound;

Fig. 6 is a schematic for explaining a stable configuration of three-dimensional structure data of testosterone;

Fig. 7 is a schematic (No. 1) for explaining a distance between an stably positioned atom and heme iron;

Fig. 8 is a schematic (No.2) for explaining the distance between the stably positioned atom and the heme iron;

Fig. 9 is a schematic (No. 3) for explaining the distance between the stably positioned atom and the heme iron;

Fig. 10 is a schematic for explaining a position of a substrate reaction atom on two-dimensional structure data of a compound of testosterone shown in Fig. 5;

Fig. 11 is a schematic for explaining metabolism candidate information that is extracted from the two-dimensional structure data of the compound of testosterone shown in Fig. 10;

Fig. 12 is a schematic for explaining contents stored in a transformation database 207;

Fig. 13 is a schematic for explaining two-dimensional structure data of a compound of a metabolite that is obtained by applying transformation to the two-dimensional structure data of the compound of testosterone shown in Fig. 5;

Fig. 14 is a schematic for explaining a metabolite of testosterone obtained through a wet test;

Fig. 15 is a flowchart (No.1) of a procedure for supporting estimation of metabolism according to an embodiment of the present invention;

Fig. 16 is a flowchart (No.2) of the procedure for supporting the estimation of metabolism according to the embodiment of the present invention;

Fig. 17 is a flowchart (No.3) of the procedure for supporting the estimation of metabolism according to the embodiment of the present invention;

Fig. 18 is a schematic (No.1) for explaining structures of heme iron, oxygen, and a substrate (camphor) in a reaction transition state;

Fig. 19 is a schematic (No. 2) for explaining the structures of heme iron, oxygen, and the substrate (camphor) in a reaction transition state;

Fig. 20 is a schematic for explaining a concept of a reaction center of a known metabolic reaction;

Fig. 21 is a schematic for explaining a concept of a structure of a reaction portion; and

Fig. 22 is a schematic for explaining a concept of the estimation of metabolism performed by applying transformation that is created based on a known metabolic reaction relating to each type of CYP to a compound of which a metabolite is to be estimated.

DETAILED DESCRIPTION

Exemplary embodiments of the present invention will be explained below in detail with reference to the accompanying drawings.

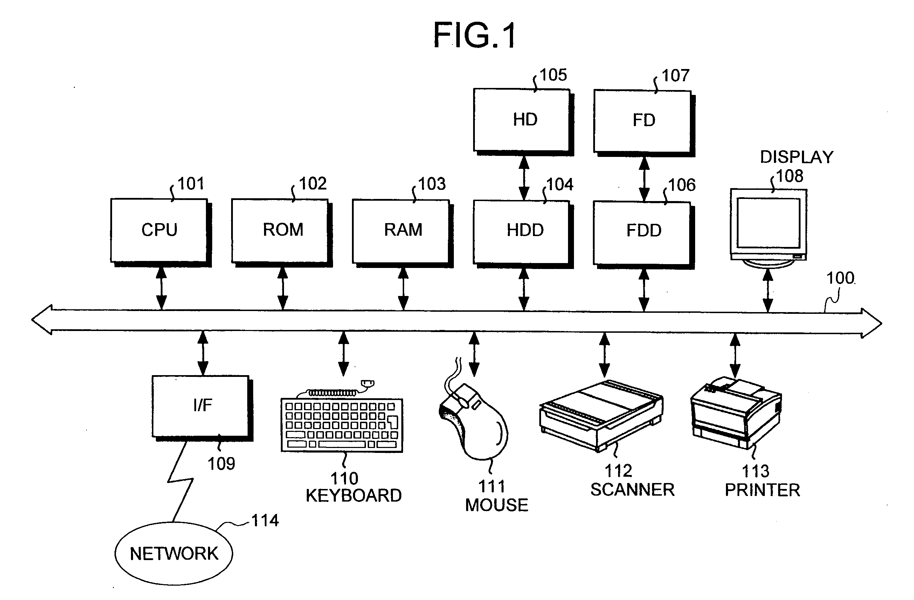

First, the hardware configuration of an apparatus for supporting estimation of metabolism according to an embodiment of the present invention is explained. Fig. 1 is a block diagram of the hardware configuration of the apparatus.

The apparatus includes a central processing unit (CPU) 101, a read only memory (ROM) 102, a random access memory (RAM) 103, a hard disk drive (HDD) 104, a hard disk (HD) 105, a flexible disk drive (FDD) 106, a flexible disk (FD) 107 as a removable recording medium, a display 108, an interface (I/F) 109, a keyboard 110, a mouse 111, a scanner 112, and a printer 113. These components are connected to each other through a bus 100.

The CPU 101 controls the apparatus. The ROM 102 stores computer programs such as a boot program. The RAM 103 is used as a work area of the CPU 101. The HDD 104 controls reading/writing of data from/to the HD 105 in accordance with the control of the CPU 101. The HD 105 stores data that is written in accordance with the control of the HDD 104.

The FDD 106 controls reading/writing of data from/to the FD 107 in accordance with the control of the CPU 101. The FD107 stores data that is written by the control of the FDD 106 and enables the apparatus for supporting estimation of metabolism to read the data stored in the FD 107.

Apart from the FD 107, a compact disc-read only memory (CD-ROM) (a compact disc-readable (CD-R), a compact disc-rewritable (CD-WR)), a magnetic optical disc (MO), a digital versatile disc (DVD), and a memory card may also be used as the removable recording medium. The display 108 displays a curser, an icon, a tool box as well as data such as documents, images, and functional information. A cathode ray tube (CRT), a thin film transistor (TFT) liquid crystal display, a plasma display can be used as the display 108.

The I/F 109 is connected to a network 114 such as the Internet through a communication line and is connected to other devices via the network 114. The I/F 109 controls the network 114 and an internal interface to control input/ output of data from external devices. A modem or a local area network (LAN) adapter can be used as the I/F 109.

The key board 110 includes keys for inputting characters, numbers, and various instructions, and is used to input data. A touch panel input pad or a numerical key pad may also be used as the key board 110. The mouse 111 is used to shift the curser, select a range, shift windows, and change sizes. A track ball or a joy stick may be used as a pointing device if similar functions are provided.

The scanner 112 optically reads an image and captures image data into the apparatus for supporting estimation of metabolism. The scanner 112 may be provided with an optical character read (OCR) function. The printer 113 prints the image data and document data. A laser printer and an inkjet printer can be used as the printer 113.

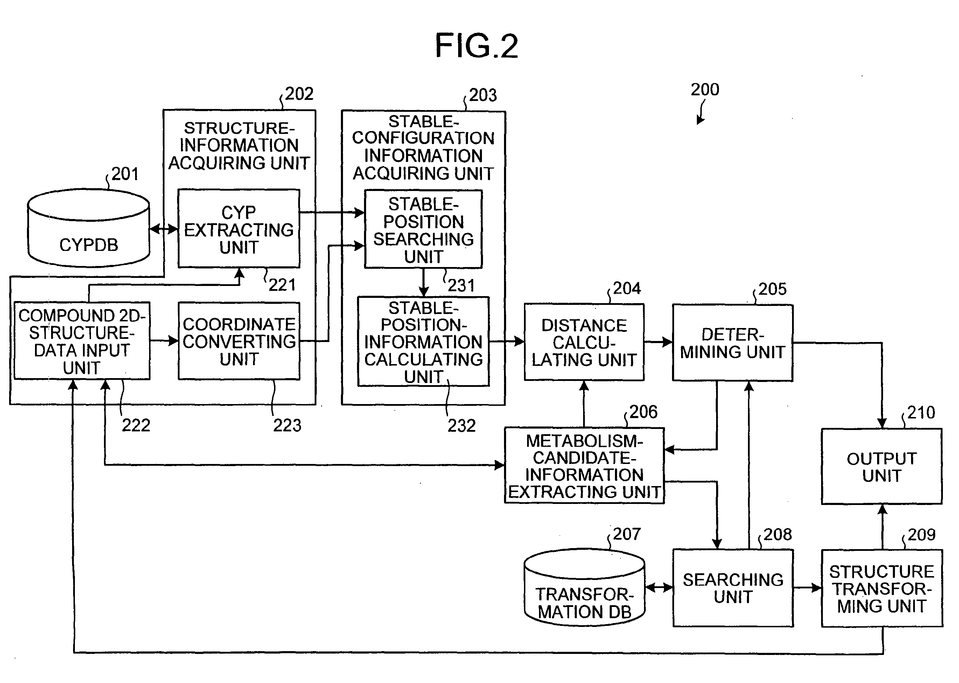

Next, the functional configuration of the apparatus is explained. Fig. 2 is a block diagram of the functional configuration of an apparatus 200 for supporting estimation of metabolism according to the embodiment. The apparatus 200 includes a CYP database (CYPDB) 201, a structure-information acquiring unit 202, a stable-configuration-information acquiring unit 203, a distance calculating unit 204, a determining unit 205, a metabolism-candidate-information extracting unit 206, a transformation database (transformation DB) 207, a searching unit 208, a structure transforming unit 209, and an output unit 210.

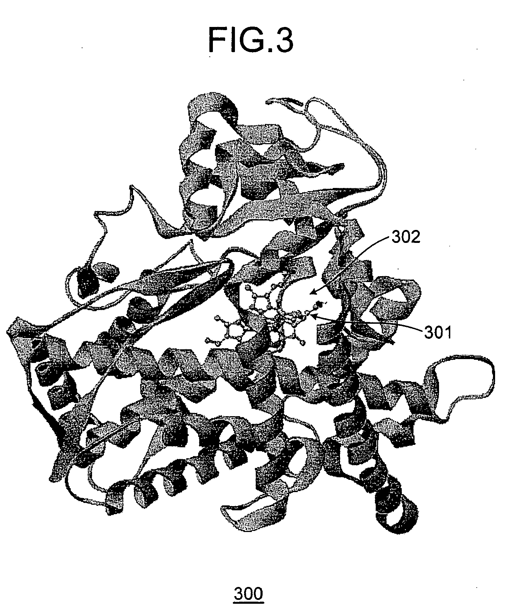

The CYP database 201 stores structure information of arbitrary types of CYP that include heme iron. The structure information of CYP is three-dimensional structure data (hereinafter, "CYP three-dimensional structure data") that includes a three-dimensional structure of CYP converted into electronic data. The CYP three-dimensional structure data includes coordinate values in predetermined three-dimensional frames of reference. The CYP three-dimensional structure data is explained taking CYP2C9 as an example. Fig. 3 is a schematic for explaining three-dimensional structure data 300 of CYP2C9.

The data 300 is obtained by X-ray crystallography. The 300 includes a heme iron 301 in the center. "Heme" is a kind of nitride that exists in a liver of a living body of humans and animals, and "heme iron" is a substance synthesized by combining heme and iron. Some types of CYP, such as CYP2C9, have a substrate bonding portion called a pocket 302 near the heme iron. The pocket 302 is a three-dimensional opening or a hollow in which a drug can be disposed.

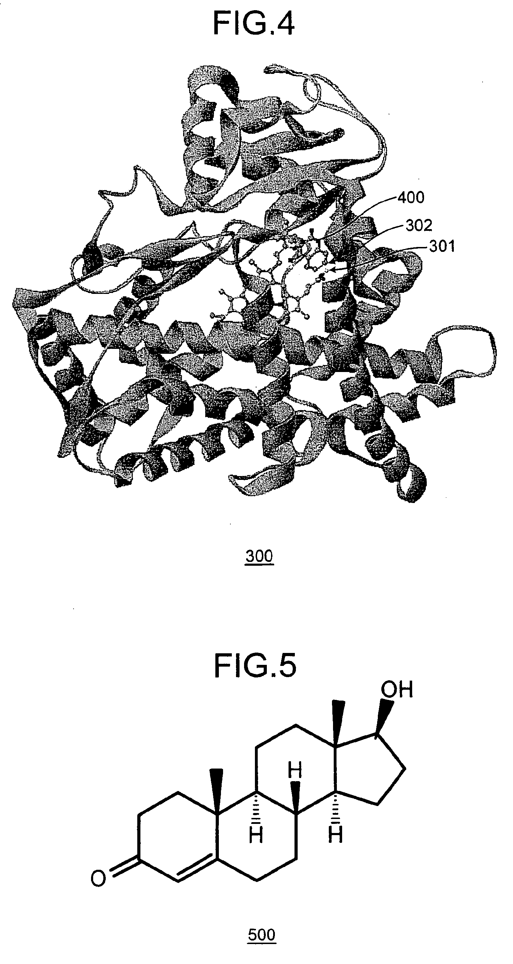

Fig. 4 is a schematic for explaining the CYP three-dimensional structure data 300 in which a drug is disposed. In the data 300 shown in Fig. 4, three-dimensional structure data 400 of S-Warfarin, which is the drug, is disposed in the pocket 302.

The CYP database 201 can be provided externally to the apparatus 200. For example, the CYP database 201 can be connected to the apparatus 200 through the network 114. Specifically, a function of the CYP database 201 is realized with a recording medium such as the ROM 102, the RAM 103, the HD 105, and the FD 107.

The structure-information acquiring unit 202 acquires structure information of an estimation target compound and arbitrary CYP three-dimensional structure data. The estimation target compound is a drug of which metabolic reaction with CYP in a living body, a metabolite that is obtained by the metabolic reaction, and an entire process of metabolism that includes several metabolic reactions are to be estimated. In addition, a metabolite that is obtained as two-dimensional structure information by the structure transforming unit 209 explained later is included.

The structure information of the estimation target compound is electronic data of the estimation target compound, and includes two-dimensional structure information (hereinafter, "compound two-dimensional structure data") that expresses a structure of the estimation target compound two-dimensionally, and three-dimensional structure information (hereinafter, "compound three-dimensional structure data") that expresses the structure of the estimation target compound three-dimensionally. The three-dimensional structure data 400 shown in Fig. 4 corresponds to the compound three-dimensional structure data.

Specifically, the compound two-dimensional structure data includes two-dimensional coordinates of each atom that constitutes the estimation target compound. Each atom includes three-dimensional scientific information that indicates a three-dimensional configuration. The three-dimensional scientific information can be converted into the compound three-dimensional structure data by a coordinate converting unit 223 explained later.

Specifically, the compound three-dimensional structure data includes three-dimensional coordinates of each atom that constitutes the estimation target compound. The compound three-dimensional structure data and the CYP three-dimensional structure data are obtained based on results from the X-ray crystallography, homology modeling, or theoretical and scientific calculation.

The structure-information acquiring unit 202 specifically includes a CYP extracting unit 221, a compound two-dimensional-structure-data input unit 222, and the coordinate converting unit 223. The CYP extracting unit 221 extracts arbitrary CYP three-dimensional structure data from the CYP database 201. Specifically, the CYP three-dimensional structure data can be extracted from the CYP database 201 when the compound two-dimensional structure data is input by the compound two-dimensional-structure-data input unit 222 explained later.

The CYP three-dimensional structure data to be extracted can be the CYP three-dimensional structure data relating to all types of CYP, or the CYP three-dimensional structure data relating to major types of CYP (1A2, 2A6, 2B6, 2C19, 2C8, 2C9, 2D6, 2E1, and 3A4). The CYP three-dimensional structure data to be extracted may also be CYP three-dimensional structure data that is designated by a user. Specifically, a function of the CYP extracting unit 221 is realized by the CPU 101 executing a computer program recorded in the ROM 102, the RAM 103, the HD 105, the FD 107, and the like, or by the I/F 109.

The compound two-dimensional-structure-data input unit 222 receives the compound two-dimensional structure data. Specifically, the compound two-dimensional structure data is input by operating the keyboard 110 and the mouse 111. The compound two-dimensional structure data may be received from an external server through the network 114 such as the Internet and the LAN. Specifically, a function of the compound two-dimensional-structure-data input unit 222 is realized with, for example, the I/F 109.

The stable-configuration-information acquiring unit 203 calculates stable position information on a stable position in which the estimation target compound is stably disposed in the pocket 302 of CYP based on the structure information of the estimation target compound and the structure information of CYP that are acquired by the structure-information acquiring unit 202. Specifically, the stable position information on the stable position in which the estimation target compound is stably disposed in the pocket 302 of CYP is acquired based on the compound three-dimensional structure data that is obtained by conversion by the coordinate converting unit 223, and the CYP three-dimensional structure data that is extracted by the CYP extracting unit 221.

A process for acquiring the stable position information is specifically explained. To metabolize a drug, the compound three-dimensional structure data should be disposed in the pocket 302 of CYP at an appropriate position and in an appropriate direction (alignment) to be oxidized. It is not necessary to put a whole structure of the drug in the pocket 302, and the drug may be disposed in such a manner that a part of the structure is in the pocket 302. A position in which the drug is thus disposed, that is a position at which the drug is stably configured, in the pocket 302 is called the stable position, and three-dimensional position coordinates of the CYP three-dimensional structure data are the stable position information.

Specifically, the stable-configuration-information acquiring unit 203 includes a stable-position searching unit 231, and a stable-position-information calculating unit 232. The stable position searching unit 231 searches the stable position described above. Specifically, the stable-position searching unit 231 searches the stable position using molecular-dynamic calculation. As the molecular-dynamic calculation, a molecular dynamics (MD) method, a molecular mechanics (MM) method, a Monte Carlo (MC) method, and the like can be used. A case in which the stable position information is calculated by the MM method is explained. Suppose a potential that is used in the MM method is an amber type potential.. The amber type potential can be calculated with formula (1):

$$E_{VDW} = \varepsilon_{ij}[(\sigma_{ij}/r)^{12} - 2(\sigma_{ij}/r)^{6}] \qquad (1)$$

In the formula (1), $E_{VDW}$ represents van der Waals energy, and r represents a distance between atoms. A parameter $\varepsilon_{ij}$ and a praramter $\sigma_{ij}$ can be calculated with formulas (2) and (3):

$$\varepsilon_{ij} = \sqrt{\varepsilon_{j} \cdot \varepsilon_{j}} \quad (kcal/mol) \qquad (2)$$

$$\sigma_{ij} = \sigma_{i} + \sigma_{j} \quad (\text{Å}) \qquad (3)$$

In the formula (2), a parameter $\varepsilon_i$ and $\varepsilon_j$ are parameters that represent strength of atoms $_i$ and $_j$ that gravitate to each other, and indicate size of a van der Waals (VDW) radius.

For a substrate atom and a protein atom, values below are substituted.

$$\varepsilon_i = \varepsilon_v = 1.00, \; \sigma_i = \sigma_v = 0.755 \; \text{(substrate atom)}$$

$$\sigma_i = \varepsilon_p = 4.25, \; \sigma_j = \sigma_p = 3.450 \; \text{(protein atom)}$$

When van der Waals energy $E_{VDW}$ is larger than a predetermined value, it is determined that the drug is in the stable position.

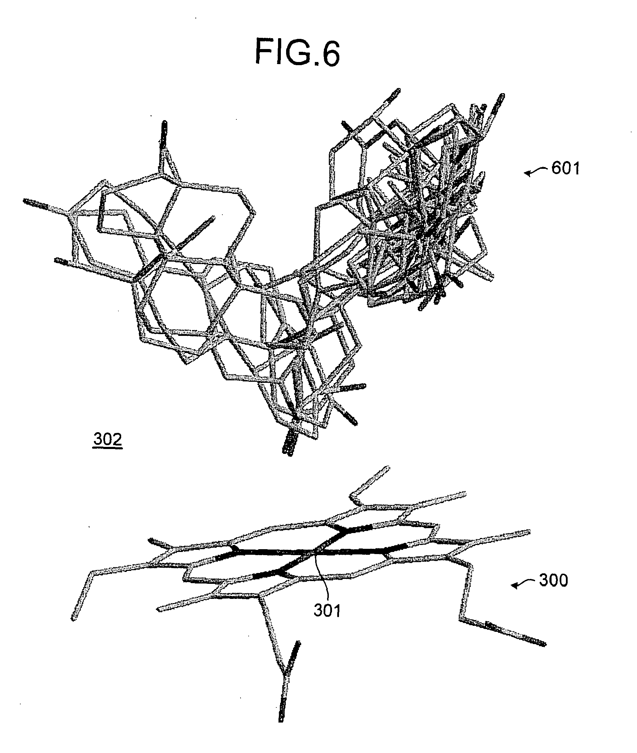

The stable position that is searched using the above formulas (1) to (3) is explained next. Fig. 5 is a schematic for explaining two-dimensional structure data (a chemical reaction formula) of testosterone, which is an estimation target compound, and Fig. 6 is a schematic for explaining a stable configuration of three-dimensional structure data of testosterone. For compound three-dimensional structure data 601 of testosterone shown in Fig. 6, the stable position that includes 12 patterns of stable positions that are constituted of a position and an alignment are searched, and the compound three-dimensional structure data 601 is disposed near the heme iron 301.

Specifically, a function of the stable-position searching unit 231 is realized by the CPU 101 executing a computer program that is stored in, for example, the ROM 102, the RAM 103, the HD 105, and the FD 107.

The stable-position-information calculating unit 232 calculates the stable position information of the drug that is disposed in the stable position. Specifically, the stable-position-information calculating unit 232 calculates the stable position information by converting three-dimensional coordinate values in the compound three-dimensional structure data 601 of the drug disposed in the stable position into three-dimensional frames of reference of CYP. Specifically, a function of the stable-position-information calculating unit 232 is realized by the CPU executing a computer program that is stored in, for example, the ROM 102, the RAM 103, the HD 105, and the FD 107.

The distance calculating unit 204 calculates a distance between an atom (hereinafter, "stably positioned atom") in the estimation target compound that is disposed in the stable position in the pocket 302 and the heme iron 301 of CYP based on the stable position information that is obtained by the stable-configuration-information acquiring unit 203. Specifically, the distance is calculated using three-dimensional coordinate values of the stably positioned atom and the heme iron 301. Specifically, a function of the distance calculating unit 204 is realized by the CPU 101 executing a computer program that is stored in, for example, the ROM 102, the RAM 103, the HD 105, and the FD 107.

A determining unit 205 determines whether the estimation target compound (more specifically, the stably positioned atom) is to be the substrate of CYP based on the distance calculated by the distance calculating unit 204. Specifically, when the distance calculated by the distance calculating unit 204 is lower than a predetermined threshold, it is determined that the estimation target compound is to be the substrate of CYP. On the other hand, when the distance calculated by the distance calculating unit 204 is not lower than the predetermined threshold, it is determined that the estimation target compound is to be the inhibitor of CYP. The threshold is determined referring to a structure of heme iron-oxygen-substrate (the stably positioned atom) in a transition state.

Determination results obtained by the determining unit 205 are explained. Suppose that the threshold described above is 5.0 [Å]. Therefore, when the distance calculated by the distance calculating unit 204 is less than 5.0 [Å], the estimation target compound in the stable position is the substrate of CYP. In other words, it is determined that the stably positioned atom is a substrate reaction atom with which the metabolic reaction is possible.

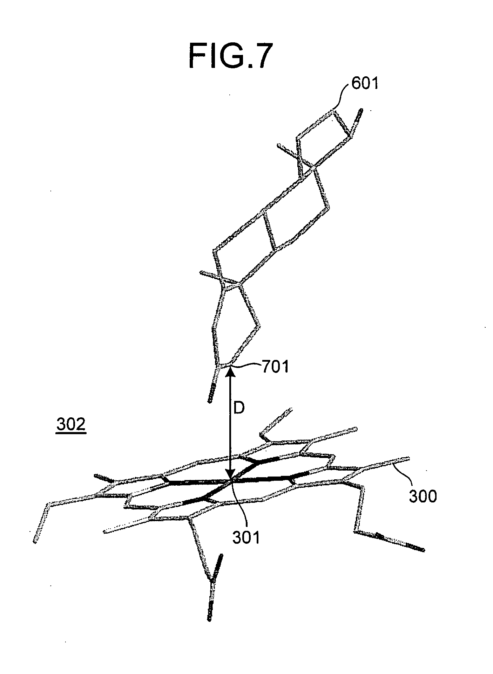

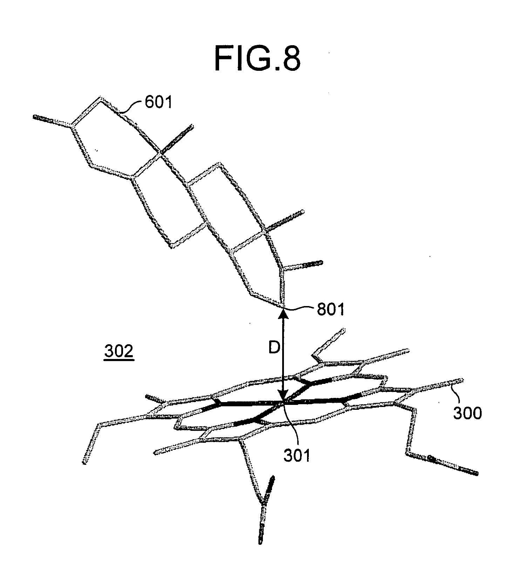

Figs. 7 to 9 are schematics for explaining the distance between the stably positioned atom and the heme iron 301. The stable positions with which the metabolic reaction is possible are extracted from among the 12 stable positions of the compound three-dimensional structure data of testosterone shown in Fig. 6, are shown in Figs. 7 to 9. A distance D between a stably positioned atom 701 in the compound three-dimensional structure data 601 of testosterone shown in Fig. 7 in the stable configuration and the heme iron 301 is 4.34 [Å]. A distance D between a stably positioned atom 801 in the compound three-dimensional structure data 601 of testosterone shown in Fig. 8 in the stable configuration and the heme iron 301 is 4.57 [Å].

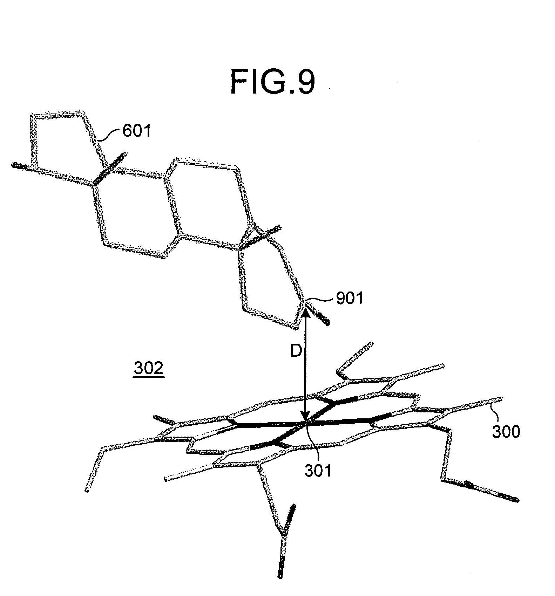

A distance D between a stably positioned atom 901 in the compound three-dimensional structure data 601 of testosterone shown in Fig. 9 in the stable configuration and the heme iron 301 is 4.88 [Å]. The stably positioned atoms 701 to 901 shown in Figs. 7 to 9 are stably positioned within a distance of 5.0 [Å] from the heme iron 301. Therefore, the stably positioned atoms 701 to 901 are substrate reaction atoms with which the metabolic reaction is possible.

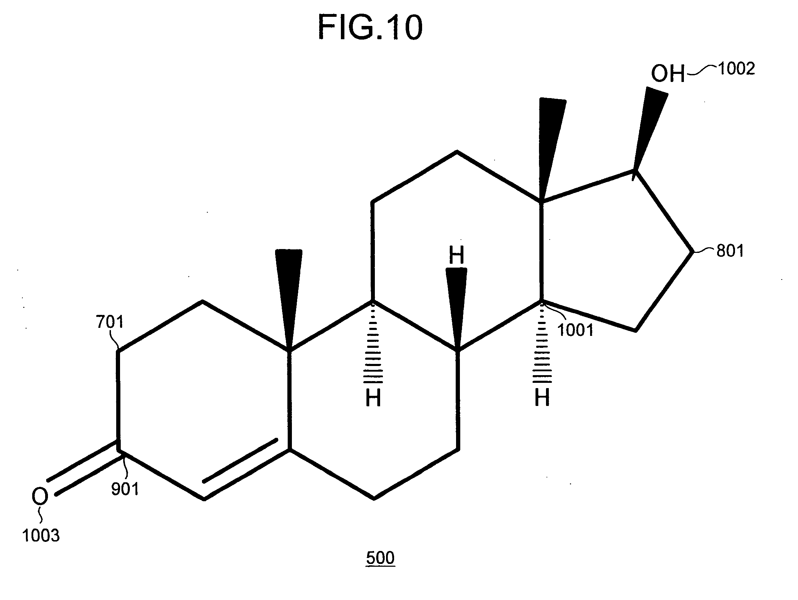

Fig. 10 illustrates a position of the substrate reaction atom in the compound two-dimensional structure data 500 of testosterone shown in Fig. 5. Fig. 10 is a schematic for explaining the position of the substrate reaction atom in the compound two-dimensional structure data of testosterone shown in Fig. 5. As shown in Fig. 10, there are six substrate reaction atoms (701, 801, 901, 1001 to 1003) that are positioned within the distance of 5.0 [Å] from the heme iron 301 among testosterone that is disposed in the 12 stable positions.

In six other stable positions in the compound three-dimensional structure data of testosterone, the distances between each of the stably positioned atoms and the heme iron 301 are more than 5.0 [Å], therefore, the stably positioned atoms of the six other stable positions inhibit metabolic reaction with the heme iron 301. Specifically, a function of the determining unit 205 is realized by the CPU 101 executing a computer program that is stored in, for example, the ROM 102, the RAM 103, the HD 105, and the FD 107.

The metabolism-candidate-information extracting unit 206 extracts metabolism candidate information relating to a metabolism candidate portion that includes an atom stably positioned in the pocket 302 from the structure information of the estimation target compound estimated to be the substrate by the determining unit 205. Specifically, the metabolism-candidate-information extracting unit 206 extracts the metabolism candidate information from the compound two-dimensional structure data 500 of the estimation target compound that includes the substrate reaction atom.

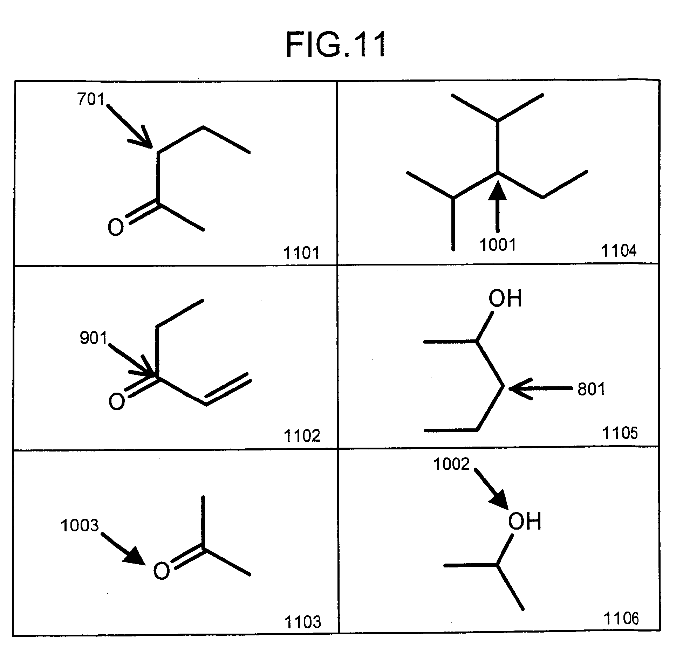

The "metabolism candidate portion" is a partial structure obtained by extracting an atom that can be traced within the predetermined bonding number from centers, which are the atoms stably positioned in the pocket 302. The atoms stably positioned are substrate reaction atoms 701, 801, 901, and 1001 to 1003. Moreover, the metabolism candidate information is two-dimensional structure data of the metabolism candidate portion. Fig. 11 is a schematic for explaining metabolism candidate information that is extracted from the compound two-dimensional structure data 500 of testosterone shown in Fig. 10.

In the case shown in Fig. 11, the predetermined bonding number is "two", and metabolism candidate information 1101 to 1106 that can be traced from the substrate reaction atoms 701, 801, 901, and 1101 to 1103 within the bonding number two respectively is extracted for each of the substrate reaction atoms 701, 801, 901, and 1101 to 1103. Specifically, a function of the metabolism-candidate-information extracting unit 206 is realized by the CPU 101 executing a computer program that is stored in, for example, the ROM 102, the RAM 103, the HD 105, and the FD 107.

The transformation DB 207 stores transformation (or a "transformation formula") that includes structure information of a reaction portion of a known substrate and structure information of a reaction portion of a known reaction product. The transformation DB 207 is a database that is constituted of the transformation that is created based on the known metabolic reaction relating to each CYP shown in Fig. 22.

In other words, in the transformation DB 207, the transformation ((c) and (d) in Fig. 22) that is created by extracting the atoms that can be traced from the reaction center atoms, which are obtained by comparing the structure of the known substrate and the structure of the known reaction product, within the predetermined bonding number (the bonding number in an example shown in Fig. 22 is one) as shown in Fig. 22.

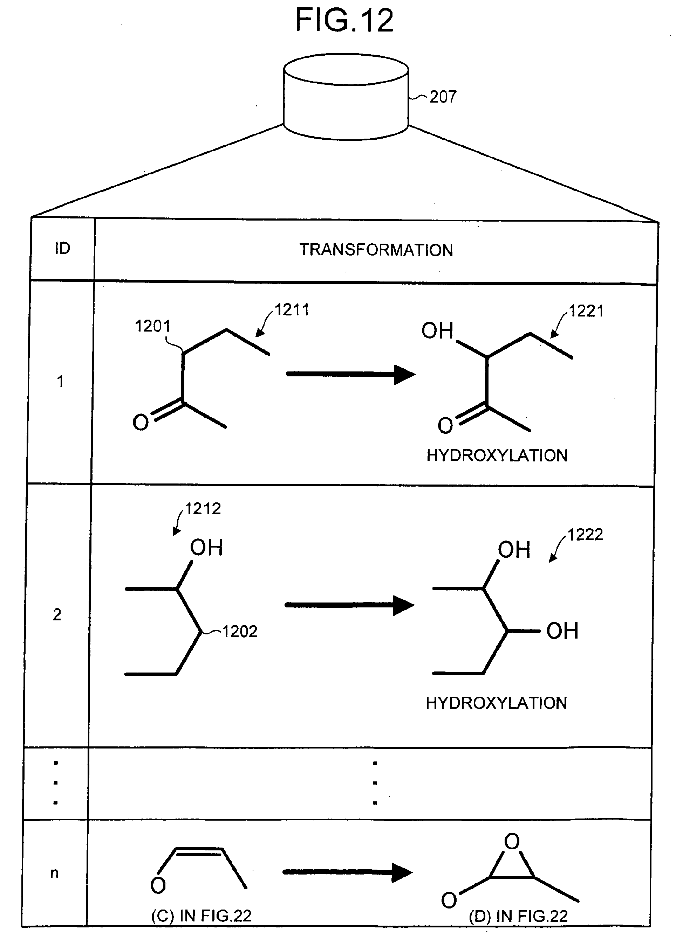

Fig. 12 is a schematic for explaining contents stored in the transformation DB 207. In Fig. 12, structure information of a reaction portion on a left side (side of a beginning end of an arrow) is the structure information of the reaction portion of the know substrate, and structure information of a reaction product on a right side (side of an pointing end of the arrow) is the structure information of the reaction portion of the know reaction product.

For example, in transformation of which ID=1, structure information 1211 of a reaction portion that includes a reaction center atom 1201 that corresponds to the substrate reaction atom is transformed to a structure in structure information 1221 of a reaction portion of a known reaction product. In a similar manner, transformation of which ID=2 structure information 1212 of a reaction portion that includes a reaction center atom 1202 that corresponds to the substrate reaction atom is transformed to a structure in structure information 1222 of a reaction portion of a known reaction product. Specifically, a function of the transformation DB 207 is realized with, for example, the ROM 102, the RAM 103, the HD 105, and the FD 107.

The searching unit 208 searches the transformation DB 207 for transformation that includes structure information of a reaction portion of a known substrate that matches the metabolism candidate information 1101 to 1106 extracted by the metabolism-candidate-information extracting unit 206. For example, the metabolism candidate information 1101 shown in Fig. 11 matches the structure information 1211 of the reaction portion of the known substrate. Therefore, the transformation of ID=1 can be searched. The metabolism candidate information 1105 matches the structure information 1211 of the reaction portion of the known substrate. Therefore, the transformation of ID=2 can be searched.

On the other hand, for the rest of the metabolism candidate information, which are metabolism candidate information 1102 to 1104, and 1106, transformation cannot be obtained because the metabolism candidate information 1102 to 1104, and 1106 do not match with the structure information of the reaction portion of the known substrate in the transformation DB 207. Thus, the metabolism candidate information 1102 to 1104, and 1106 for which transformation cannot be obtained are determined to be portions that inhibit metabolism of CYP, Specifically, a function of the searching unit 208 is realized by the CPU 101 executing a computer program that is stored in, for example, the ROM 102, the RAM 103, the HD 105, and the FD 107, or by the I/F 109.

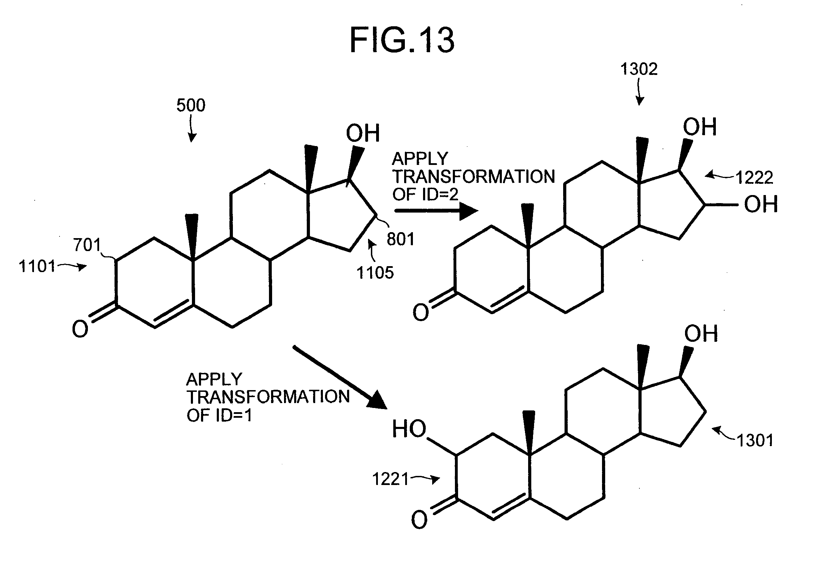

The structure transforming unit 209 transforms (applies the transformation) the metabolism candidate information 1101, and 1105 to the a structure in structure information 1121, and 1222 of the reaction portion of the known reaction product in the transformation searched by the searching unit 208 Fig. 13 is a schematic for explaining compound two-dimensional structure data of a metabolite that is obtained by applying transformation to the compound two-dimensional structure data of testosterone shown in Fig. 5.

Compound two-dimensional structure data 1301 shown in Fig. 13 is two-dimensional structure data of a metabolite that is obtained by applying the transformation of ID=1 shown in Fig. 12. Compound two-dimensional structure data 1302 shown in Fig. 13 is two-dimensional structure data of a metabolite that is obtained by applying the transformation of ID=2 shown in Fig. 12.

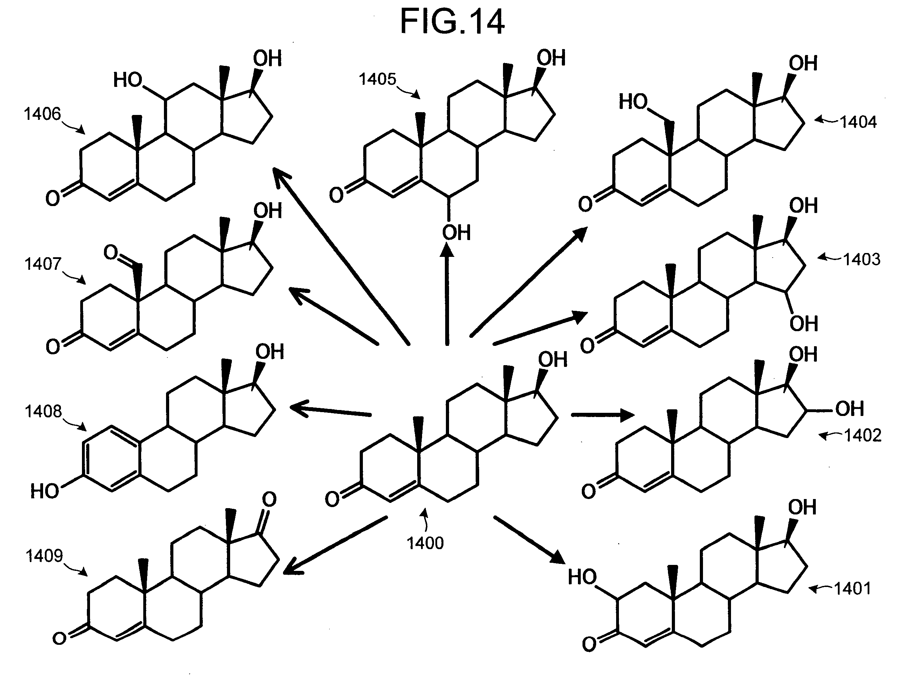

A metabolite of testosterone that is obtained through a wet test is explained. Fig. 14 is a schematic for explaining the metabolite of testosterone obtained through the wet test. It took a few months to obtain this result. As shown in Fig. 14, there are nine compounds (1401 to 1409) that are similar in structure to testosterone 1400. Among the nine compounds shown in Fig. 14, metabolites that are determined to be generated by metabolism of the testosterone 1400 by an actual experiment are metabolites 1401 to 1403, and 1405. The metabolites 1401 to 1403, and 1405 are compared with the compound two-dimensional structure data 1301, and 1302 of the metabolite that are obtained by the structure transforming unit 209.

The compound two-dimensional structure data 1301 and 1302 completely match with the metabolite 1401 and 1402 obtained in the actual experiment shown in Fig. 14, and this proves that the metabolites are accurately estimated. With the apparatus for supporting estimation of metabolism 200, it is possible to obtain the compound two-dimensional structure data 1301, and 1302 in a few days. Thus, it is possible to carry out accurate estimation in a short time. Specifically, a function of the structure transforming unit 209 is realized by the CPU 101 executing a computer program that is stored in, for example, the ROM 102, the RAM 103, the HD 105, and the FD 107.

The output unit 210 outputs the compound two-dimensional structure data 1301 and 1302 that are obtained base on determination result obtained by the determining unit 205, and by applying transformation by the structure transforming unit 209. Output may be done by displaying on the display 108, or by printing out by the printer 113. It may be output by transmitting to an external computer through the network 114. Specifically, a function of the output unit 210 is realized by the CPU 101 executing a computer program that is stored in, for example, the ROM 102, the RAM 103, the HD 105, and the FD 107, or by the I/F 109.

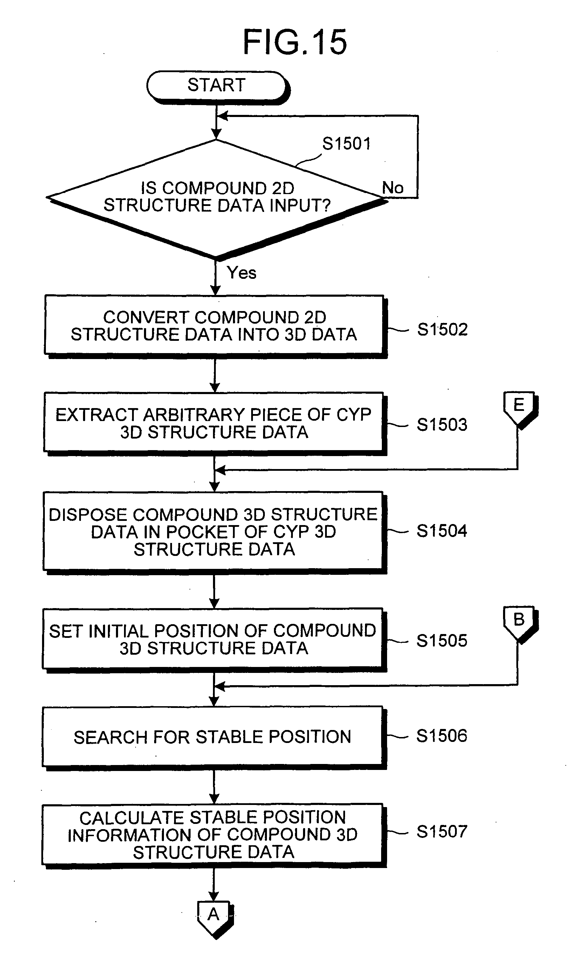

Next, a procedure of supporting estimation of metabolism according to an embodiment of the present invention is explained. Figs. 15 to 17 are flowcharts of the procedure for supporting estimation of metabolism according to the embodiment of the present invention. As shown in Fig. 15, first, when compound two-dimensional structure data is input (step S1501 : Yes), the coordinate converting unit 223 converts the compound two-dimensional structure data, which is input, into three-dimensional data (step S1502).

Moreover, the CYP extracting unit 221 extracts an arbitrary piece of CYP three-dimensional structure data 300 from the CYP database 201 (step S1503), and configures the compound three-dimensional structure data 601 that are converted into the three-dimensional data at step S1502 in the pocket 302 in the CYP three-dimensional structure data 300 (step S1504). Configuration does not have to be highly accurate.

Then, the stable-position searching unit 231 sets three-dimensional coordinates of the compound three-dimensional structure data 601, which is not precisely configured, as an initial position (step S1505), and searches for a stable position (step S1506). Search for the stable position is carried out by randomly shifting a position of the compound three-dimensional structure data 601 configured in the pocket 302. When the van der Waals energy in the formula (1) described above becomes higher than a predetermined value, a position is determined to be the stable position.

Then, the stable-position-information calculating unit 232 calculates, for the compound three-dimensional structure data 601 that are configured in the stable position, stable position information, which is to be three-dimensional coordinate values of a center atom and each atom that constitutes the compound three-dimensional structure data 601, using the three-dimensional coordinates of the initial position acquired at step S1505 (step S1507). Then, it is determined whether stable position information that is substantially identical exists (step S1601). Whether the stable position information is substantially identical is determined by comparing the stable position information calculated this time and stable position information that has already been calculated. When the three-dimensional coordinate values are identical, or a difference between the three-dimensional coordinate values is a few [Å], for example, 0.1 [Å], as a result of the comparison, it is determined to be substantially identical.

When it is determined to be substantially identical (step S1601: Yes), the stable position information is compiled as identical stable position information (step S1602). Specifically, it is compiled by deleting the stable position information of this time, or by deleting the stable position information with which the stable position information of this time is compared. The stable position information may be compiled by taking an average of pieces of the stable position information that are substantially identical. Thus, increase in the number of the stable position information can be avoided, thereby speeding up a calculating process.

On the other hand, when it is determined not to be substantially identical (step S1601: No), the stable position information is stored as a new piece of stable position information (step S1603). Then, it is determined whether acquisition of the stable position information by the stable-position-information acquiring unit 203 is finished (step S1604). Specifically, the acquisition of the stable position information is finished when no stable position information is searched by the stable-position searching unit 231, when the number of trial of searching reaches a predetermined number, when a predetermined time limit has passed, or when a user inputs termination of the acquisition.

When the acquisition of the stable position information is not finished (step S1604: No), a process proceeds to step S1506 shown in Fig. 15. On the other hand, when the acquisition of the stable position information is finished (step S1605: Yes), the distance calculating unit 204 calculates a distance between a stably positioned atom and heme iron with respect to each of the stable positions (step S1605).

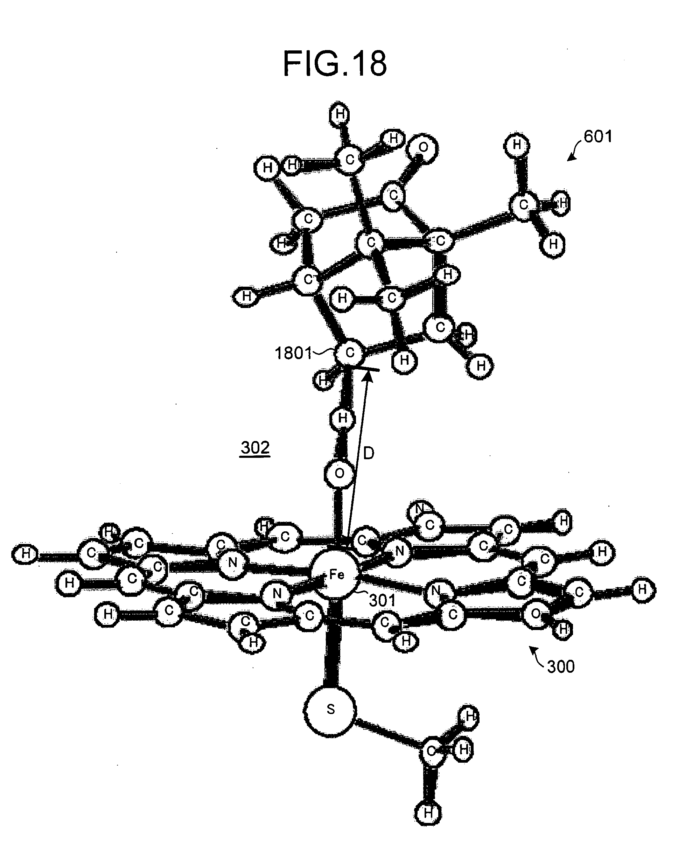

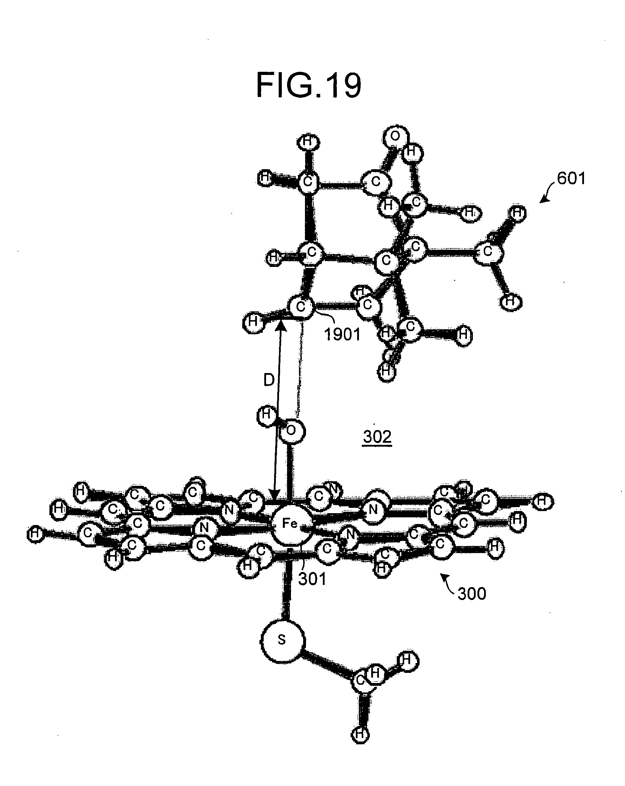

Then, the determining unit 205 determines whether there is a distance that is less than a predetermined threshold among the distances calculated (step S1606). The distance is explained with reference to structures of heme iron, oxygen, and a substrate (camphor) in a reaction transition state that are acquired by a theoretical and scientific calculation by Yoshizawa et al. Figs. 18 and 19 are schematics for explaining the structures of heme iron, oxygen, and a substrate (camphor) in the reaction transition state. A distance D between a stably positioned atom 1801 (or 1901) and the heme iron 301 shown in Figs. 18 and 19 is 4.4 [Å] to 5.1 [Å], and this extent of distance is appropriate for the predetermined threshold.

When it is determined that there is no distance that is less than the predetermined threshold (step S1606: No), the output unit 210 outputs information that indicates possibility of the estimation target compound being the inhibitor (step S1607).

After this process is done, if there is no CYP three-dimensional structure data left without being extracted from the CYP database 201 (step S1608: No), a series of the process is completed. On the other hand, if there is CYP three-dimensional structure data left without being extracted from the CYP database 201 (step S1608: Yes), the dimensional structure data that has not been extracted is extracted (step S1609), and the process proceeds to step S1504.

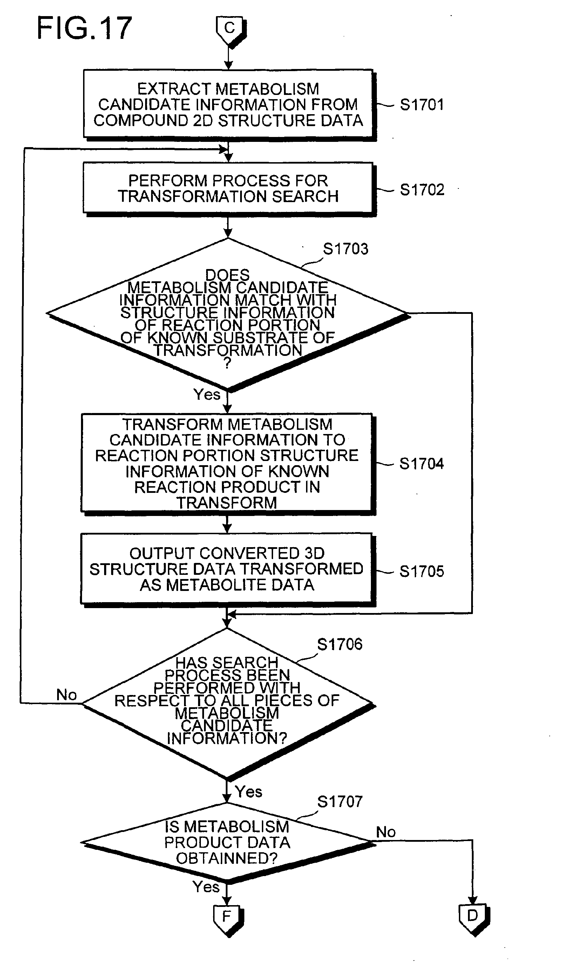

Moreover, when it is determined, at step S1606, that there is a distance that is less than the threshold among the distances calculated by the distance calculating unit 204 (step S1606: Yes), the metabolism candidate information is extracted form the compound two-dimensional structure data (step S1701) as shown in Fig. 17, a search process for transformation is performed (step S1702). In the search process, transformation that includes the structure information of the reaction portion of the known substrate that matches the metabolism candidate information extracted at step S1701.

When the metabolism candidate information does not match with the structure information of the reaction portion of the known substrate in the transformation (step S1703: No), the process proceeds to step S1706. On the other hand, when the metabolism candidate information matches with the structure information of the reaction portion of the known substrate (step S1703: Yes), the structure transforming unit 209 transforms the metabolism candidate information to the structure of the reaction portion of the known reaction product in the transformation (step S1704). Then, the output unit 210 outputs the compound three-dimensional data transformed as metabolite data (step S1705).

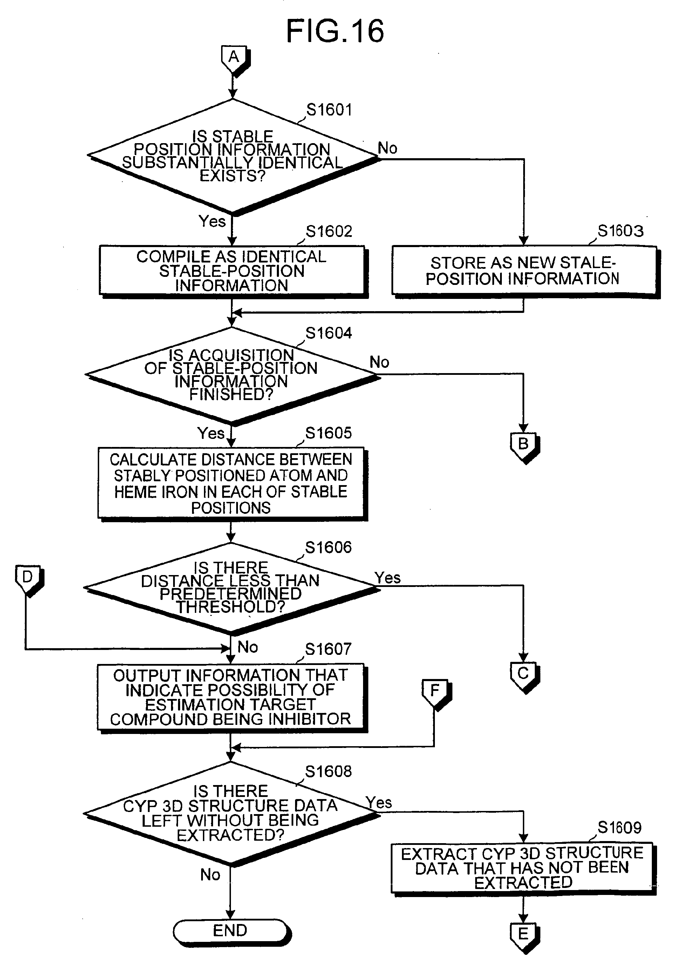

At step S1706, if the search process has not been performed with respect to all pieces of the metabolism candidate information (step S1706: No), the process proceeds to step S1702. On the other hand, if the search process has been performed with respect to all pieces of the metabolism candidate information (step S1706: Yes), the determining unit 205 determines whether the metabolite data is obtained (step S1707). When the metabolite data is not obtained (step S1707: No), the process proceeds to step S1607 shown in Fig. 16, and the output unit 210 outputs the information that indicates the possibility of the estimation target compound being the inhibitor. On the other hand, when the metabolite data is obtained (step S1707: Yes), the process proceeds to step S1608 shown in Fig. 16.

The metabolite data obtained has two-dimensional structure data of a metabolite of a first degree (hereinafter, "first metabolite"). It is possible to determine whether the first metabolite is to be the substrate or the inhibitor of CYP by inputting the two-dimensional structure data of the first metabolite as a new piece of compound two-dimensional structure data, and by executing the processes shown in Figs. 15 to 17. When it is determined to be the substrate, it is possible to obtain two-dimensional structure data of a second metabolite by applying the transformation described above to the two-dimensional structure data of the first metabolite.

With such procedure, it is possible to obtain two-dimensional structure data of Nth metabolite (where N is a natural number). In other words, if the two-dimensional structure data of the first to Nth metabolites (where N is a natural number) are two-dimensional structure data of an excretable substance, such as water and oxygen, it is possible to judge safety of the metabolic process in a living body with high accuracy, and to apply for the estimation of the metabolic reaction, the metabolite, and the metabolic process.

Thus, with the apparatus for supporting estimation of metabolism 200 and the method for supporting estimation of metabolism according to the present embodiment, it is possible to improve determination accuracy in the estimation of the metabolic reaction by estimating bonding possibilities with respect to the transformation of the estimation target compound (including the Nth metabolite) that is determined to be the substrate of CYP.

Furthermore, a portion at which bonding is possible is obtained by the transformation, and it is possible to provide the Nth metabolite obtained to users as a metabolite candidate with high accuracy on grounds that a real-world example is obtained in an environment around the portion. Moreover, it is possible to search the process of the metabolism, thereby enabling evaluating selectivity among plural types of CYP, or idiosyncracy of CYP to one substrate.

As explained above, according to the apparatus, the method, and the computer program for supporting estimation of metabolism and the recording medium according to the present invention, it is possible to support efficient estimation of effective metabolic reaction for a drug, a metabolite, and a metabolic process for a drug with high accuracy using massive data relating to CYP. Furthermore, this contributes to decreasing the number of drops due to the drug metabolism in clinical trials in the drug development, thereby shortening a developing period and decreasing developing cost.

The method for supporting estimation of metabolism explained in the present embodiment can be implemented by executing a computer program, which is prepared in advance, with a computer, such as a personal computer and a workstation. The computer program is stored in a computer-readable recording medium, such as a compact disc-read only memory, a magneto optical disk, and a digital versatile disk, and is executed by being read by the computer from such recording medium. The computer program may be a transmission medium that can be distributed through a network such as the Internet.

With the apparatus, the method, and the computer program for supporting estimation of metabolism according to the present invention, it is possible to support efficient estimation of effective metabolic reaction for a drug, a metabolite, and a process of metabolism for the drug with high accuracy using massive data relating to CYP.

Although the invention has been described with respect to a specific embodiment for a complete and clear disclosure, the appended claims are not to be thus limited but are to be construed as embodying all modifications and alternative constructions that may occur to one skilled in the art that fairly fall within the basic teaching herein set forth.

**Claims**

1. An apparatus for supporting estimation of metabolism, comprising:

     a structure-information acquiring unit that acquires structure information of an estimation target compound and structure information of an arbitrary unit of cytochrome that includes heme iron;
     a stable-position-information acquiring unit that acquires stable position information that includes information on a stable position at which the estimation target compound is stably disposed in a pocket of the cytochrome based on the structure information of the estimation target compound and the structure information of the cytochrome that are acquired by the structure-information acquiring unit;
     a distance calculating unit that calculates a distance between an atom that is stably disposed in the estimation target compound in the pocket and the heme iron of the cytochrome based on the stable position information acquired by the stable-position-information acquiring unit; and
     a determining unit that determines whether the estimation target compound is to be a substrate of the cytochrome based on at least the distance calculated by the distance calculating unit.

2. The apparatus according to claim 1, wherein, when the distance calculated by the distance calculating unit is less than a predetermined threshold, the determining unit determines that the estimation target compound is to be the substrate of the cytochrome.

3. The apparatus according to claim 1 or 2, wherein, when the distance calculated by the distance calculating unit is not less than a predetermined threshold, the determining unit determines that the estimation target compound is to be the substrate of the cytochrome.

4. The apparatus according to claim 1, 2 or 3, further comprising:

     an extracting unit that extracts, from structure information of an estimation target compound that is determined to be the substrate by the determining unit, metabolism candidate information relating to a metabolism candidate portion that includes an atom stably disposed in the pocket;
     a searching unit that searches a group of transformation that includes structure information of a reaction portion of a known substrate and structure information of a reaction portion of a known reaction product, for transformation that includes structure information of a reaction portion of the known substrate that matches the metabolism candidate information extracted by the extracting unit; and
     a transforming unit that transforms the metabolism candidate information to the structure information of the reaction portion of the known reaction product.

5. The apparatus according to claim 4, wherein the determining unit determines, when the transformation that includes the structure information of the reaction portion of the known substrate that matches the metabolism candidate information is not searched by the searching unit, that the estimation target compound is to be an inhibitor of the cytochrome.

6. The apparatus according to claim 4 or 5, wherein the structure-information acquiring unit acquires structure information of a metabolite that is obtained by transformation by the transforming unit as the structure information of the estimation target compound.

7. A method for supporting estimation of metabolism, comprising:

acquiring structure information of an estimation target compound and structure information of an arbitrary unit of cytochrome that includes heme iron;
acquiring stable position information that includes information on a stable position at which the estimation target compound is stably disposed in a pocket of the cytochrome based on the structure information acquired;
calculating a distance between an atom that is stably disposed in the estimation target compound in the pocket and the heme iron of the cytochrome based on the stable position information acquired; and
determining whether the estimation target compound is to be a substrate of the cytochrome based on the distance calculated.

8. The method according to claim 7, wherein, when the distance calculated is less than a predetermined threshold, the determining includes determining that the estimation target compound is to be the substrate of the cytochrome.

9. The method according to claim 7 or 8, wherein, when the distance calculated is not less than a predetermined threshold, the determining includes determining that the estimation target compound is to be the substrate of the cytochrome.

10. The method according to claim 7, 8 or 9, further comprising:

extracting, from structure information of an estimation target compound that is determined to be the substrate, metabolism candidate information relating to a metabolism candidate portion that includes an atom stably disposed in the pocket;
searching a group of transformation that includes structure information of a reaction portion of a known substrate and structure information of a reaction portion of a known reaction product, for transformation that includes structure information of a reaction portion of the known substrate that matches the metabolism candidate information extracted; and
transforming the metabolism candidate information to the structure information of the reaction portion of the known reaction product.

11. The method according to claim 10, wherein the determining includes determining, when the transformation that includes the structure information of the reaction portion of the known substrate that matches the metabolism candidate information is not found at the searching, that the estimation target compound is to be an inhibitor of the cytochrome.

12. The method according to claim 10 or 11, wherein the acquiring the structure-information includes acquiring structure information of a metabolite that is obtained by transformation at the transforming as the structure information of the estimation target compound.

13. A computer program for realizing a method for supporting estimation of metabolism on a computer, the computer program causing the computer to execute:

acquiring structure information of an estimation target compound and structure information of an arbitrary unit of cytochrome that includes heme iron;
acquiring stable position information that includes information on a stable position at which the estimation target compound is stably disposed in a pocket of the cytochrome based on the structure information acquired;
calculating a distance between an atom that is stably disposed in the estimation target compound in the pocket and the heme iron of the cytochrome based on the stable position information acquired; and
determining whether the estimation target compound is to be a substrate of the cytochrome based on the distance calculated.

14. A computer-readable recording medium that stores a computer program for realizing a method for supporting estimation of metabolism on a computer, the computer program causing the computer to execute:

acquiring structure information of an estimation target compound and structure information of an arbitrary unit of cytochrome that includes heme iron;

acquiring stable position information that includes information on a stable position at which the estimation target compound is stably disposed in a pocket of the cytochrome based on the structure information acquired;

calculating a distance between an atom that is stably disposed in the estimation target compound in the pocket and the heme iron of the cytochrome based on the stable position information acquired; and

determining whether the estimation target compound is to be a substrate of the cytochrome based on the distance calculated.

# FIG.1

EP 1 632 577 A2

# FIG.2

EP 1 632 577 A2

# FIG.3

302

301

300

# FIG.4

400
302
301

300

# FIG.5

500

# FIG.6

302

601

301

300

# FIG.7

# FIG.8

# FIG.9

# FIG.10

500

EP 1 632 577 A2

# FIG.11

# FIG.12

| ID | TRANSFORMATION |
|---|---|
| 1 | HYDROXYLATION |
| 2 | HYDROXYLATION |
| . . . | . . . |
| n | (C) IN FIG.22 → (D) IN FIG.22 |

FIG.13

APPLY TRANSFORMATION OF ID=2

APPLY TRANSFORMATION OF ID=1

500

701

1101

801

1105

1302

1222

1301

1221

FIG.14

EP 1 632 577 A2

# FIG.15

```
          ┌─────────────┐
          │    START    │
          └─────────────┘
                 │
                 ▼
              ╱─────╲         S1501
            ╱         ╲
          ╱ IS COMPOUND ╲    No
        ╱  2D STRUCTURE   ╲─────────┐
          ╲ DATA INPUT?  ╱          │
            ╲         ╱              │
              ╲─────╱                │
                 │ Yes              │
                 ▼                  │
   ┌──────────────────────────┐     │
   │   CONVERT COMPOUND 2D     │     │
   │ STRUCTURE DATA INTO 3D    │── S1502
   │          DATA            │     │
   └──────────────────────────┘     │
                 │                  │
                 ▼                  │
   ┌──────────────────────────┐    ┌─┐
   │ EXTRACT ARBITRARY PIECE  │    │E│
   │ OF CYP 3D STRUCTURE DATA │── S1503
   └──────────────────────────┘     │
                 │◄─────────────────┘
                 ▼
   ┌──────────────────────────┐
   │ DISPOSE COMPOUND 3D      │
   │ STRUCTURE DATA IN POCKET │── S1504
   │ OF CYP 3D STRUCTURE DATA │
   └──────────────────────────┘
                 │
                 ▼
   ┌──────────────────────────┐    ┌─┐
   │ SET INITIAL POSITION OF  │    │B│
   │ COMPOUND 3D STRUCTURE    │── S1505
   │          DATA            │     │
   └──────────────────────────┘     │
                 │◄─────────────────┘
                 ▼
   ┌──────────────────────────┐
   │ SEARCH FOR STABLE        │── S1506
   │       POSITION           │
   └──────────────────────────┘
                 │
                 ▼
   ┌──────────────────────────┐
   │ CALCULATE STABLE         │
   │ POSITION INFORMATION OF  │── S1507
   │ COMPOUND 3D STRUCTURE    │
   │          DATA            │
   └──────────────────────────┘
                 │
                 ▼
               ┌─┐
               │A│
               └─┘
```

# FIG.16

A

**S1601**

IS STABLE
POSITION INFORMATION
SUBSTANTIALLY IDENTICAL
EXISTS?

No

Yes

**S1602**

COMPILE AS IDENTICAL
STABLE-POSITION
INFORMATION

**S1603**

STORE AS NEW STALE-
POSITION INFORMATION

**S1604**

IS ACQUISITION
OF STABLE-POSITION
INFORMATION
FINISHED?

No

Yes

**S1605**

CALCULATE DISTANCE BETWEEN
STABLY POSITIONED ATOM AND
HEME IRON IN EACH OF STABLE
POSITIONS

B

**S1606**

D

IS THERE
DISTANCE LESS THAN
PREDETERMINED
THRESHOLD?

Yes

No

**S1607**

OUTPUT INFORMATION THAT
INDICATE POSSIBILITY OF
ESTIMATION TARGET
COMPOUND BEING INHIBITOR

F

C

**S1608**

IS THERE
CYP 3D STRUCTURE DATA
LEFT WITHOUT BEING
EXTRACTED?

Yes

No

**S1609**

EXTRACT CYP 3D STRUCTURE
DATA THAT HAS NOT BEEN
EXTRACTED

END

E

# FIG.17

C

EXTRACT METABOLISM
CANDIDATE INFORMATION FROM
COMPOUND 2D STRUCTURE DATA — S1701

PERFORM PROCESS FOR
TRANSFORMATION SEARCH — S1702

S1703

DOES
METABOLISM CANDIDATE
INFORMATION MATCH WITH
STRUCTURE INFORMATION
OF REACTION PORTION
OF KNOWN SUBSTRATE OF
TRANSFORMATION
?

Yes

TRANSFORM METABOLISM
CANDIDATE INFORMATION TO
REACTION PORTION STRUCTURE
INFORMATION OF KNOWN
REACTION PRODUCT IN
TRANSFORM — S1704

OUTPUT CONVERTED 3D
STRUCTURE DATA TRANSFORMED
AS METABOLITE DATA — S1705

S1706

HAS SEARCH
PROCESS BEEN
PERFORMED WITH
No — RESPECT TO ALL PIECES OF
METABOLISM
CANDIDATE
INFORMATION?

Yes

S1707

IS METABOLISM
PRODUCT DATA
OBTAINNED?        No

Yes

F        D

# FIG.18

# FIG.19

# FIG.20

(a)                                                    (b)

REACTION CENTER

# FIG.21

(a)                                                    (b)

STRUCTURE OF
REACTION PORTION

# FIG.22

(a)

KNOWN
METABOLIC
REACTION

(b)

CREATE TRANSFORMATION

(c)

(d)

TRANSFORMATION

APPLY
TRANSFORMATION

(e)

COMPOUND OF WHICH
METABOLITE IS TO BE
ESTIMATED

ESTIMATE
METABOLITE

(f)

ESTIMATED METABOLITE

EP 1 632 577 A2